(19) Europäisches Patentamt
European Patent Office
Office européen des brevets

(11) **EP 4 104 898 A1**

(12) **EUROPEAN PATENT APPLICATION**
published in accordance with Art. 153(4) EPC

(43) Date of publication:
**21.12.2022 Bulletin 2022/51**

(21) Application number: **20918711.1**

(22) Date of filing: **14.08.2020**

(51) International Patent Classification (IPC):
*A61N 2/02* (2006.01)    *A61N 2/00* (2006.01)
*A61K 41/00* (2020.01)    *H01F 1/00* (2006.01)

(52) Cooperative Patent Classification (CPC):
**A61K 41/00; A61N 2/00; A61N 2/02; H01F 1/00**

(86) International application number:
**PCT/KR2020/010888**

(87) International publication number:
**WO 2021/162187 (19.08.2021 Gazette 2021/33)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.02.2020 KR 20200018124**

(71) Applicant: **Seoul National University R & DB
Foundation
Seoul 08826 (KR)**

(72) Inventors:
• **KIM, Sang Koog
Seongnam-si, Gyeonggi-do 13528 (KR)**
• **LEE, Jae Hyeok
Seoul 08788 (KR)**

(74) Representative: **Marks & Clerk LLP
15 Fetter Lane
London EC4A 1BW (GB)**

(54) **MAGNETIC NANOPARTICLE HEATING METHOD USING RESONANCE**

(57)    Provided is a magnetic nanoparticle heating method using resonance, the method including (a) providing magnetic nanoparticles, (b) applying a direct current (DC) magnetic field to the magnetic nanoparticles, and (c) applying an alternating current (AC) magnetic field to the magnetic nanoparticles 100, wherein a temperature change rate dT/dt of the magnetic nanoparticles is increased to at least 10 K/s or more by adjusting at least one of a strength of the DC magnetic field, a frequency of the AC magnetic field, a strength of the AC magnetic field, and a pulse width of the AC magnetic field.

FIG. 9

(a)

**Description**

TECHNICAL FIELD

The present invention relates to a magnetic nanoparticle heating method using resonance, and more particularly, to a magnetic nanoparticle heating method using resonance, the method being capable of efficiently generating heat within a short time by controlling a factor of a direct current (DC)/alternating current (AC) magnetic field applied to magnetic nanoparticles.

BACKGROUND ART

Currently, research using various types of nanoparticles is being actively conducted in biomedical fields, e.g., cell staining, cell isolation, in-vivo drug delivery, gene delivery, disease or disorder diagnosis and treatment, and molecular imaging.

Among them, various fields for generating heat from magnetic nanoparticles and applying the generated heat are being studied. For example, hyperthermia is a technology for treating an affected area by applying heat at a temperature higher than body temperature. In general, when exposed to heat 5 °C or higher than body temperature, body tissues or cells may be killed due to protein denaturation. In particular, at a temperature of 42 °C or higher, cancer cells may be effectively killed and immune cells may be activated by the action of heat. As such, to remove tumors or cancer cells, hyperthermia may be applied alone or together with radiotherapy or anticancer therapy.

Despite the above-described advantages of hyperthermia, it is not easy to transfer heat intensively and effectively to tumors or cancer cells positioned deep inside a human body. A method of killing malignant cells of an affected area by inserting an antenna and a high-frequency electrode into a human body and then applying a high-frequency current from the outside is currently introduced.

DETAILED DESCRIPTION OF THE INVENTION

TECHNICAL PROBLEM

However, the above-described existing technology may generate heat of only up to about 1 kW/g. For example, $Fe_3O_4$ nanoparticles approved by the U.S. Food and Drug Administration (FDA) may not be applied to hyperthermia due to a low heating temperature and severe changes in crystalline, magnetic, and heating properties caused by a surrounding environment, and may not easily achieve a value of 2 kW/g which is ideal for the treatment of tumors having a radius of 10 mm or more.

Existing methods of generating heat from magnetic nanoparticles are based on a principle of generating heat by using energy due to magnetic hysteresis loss caused by the application of high-frequency current, or generating heat due to Brownian relaxation. To this end, a very strong magnetic field of several hundreds of Oe or more needs to be applied, which leads to a high cost and a large size of an apparatus.

Furthermore, the existing hyperthermia methods additionally require physical surgery to insert an antenna and a high-frequency electrode into a human body. In addition, it is not easy to accurately specify a target area for hyperthermia and thus not only tumors and cancer cells but also surrounding normal tissues are killed.

The present invention provides a magnetic nanoparticle heating method capable of more efficiently generating heat from magnetic nanoparticles.

The present invention also provides a magnetic nanoparticle heating method capable of generating high heat by applying a low magnetic field.

The present invention also provides a magnetic nanoparticle heating method capable of achieving a low cost and a small size of an apparatus.

The present invention also provides a magnetic nanoparticle heating method capable of selectively and intensively generating heat in a specific treatment area for hyperthermia.

However, the scope of the present invention is not limited thereto.

TECHNICAL SOLUTION

According to an aspect of the present invention, there is provided a magnetic nanoparticle heating method including (a) providing magnetic nanoparticles, (b) applying a direct current (DC) magnetic field to the magnetic nanoparticles, and (c) applying an alternating current (AC) magnetic field to the magnetic nanoparticles 100, wherein a temperature change rate dT/dt of the magnetic nanoparticles is increased to at least 10 K/s or more by adjusting at least one of a strength of the DC magnetic field, a frequency of the AC magnetic field, a strength of the AC magnetic field, and a pulse width of the AC magnetic field.

Step (b) may include applying the DC magnetic field to make the magnetic nanoparticles have a resonance frequency, and step (c) may include applying the AC magnetic field having a frequency equal to the resonance frequency of the magnetic nanoparticles, to exhibit a maximum value of the temperature change rate dT/dt of the magnetic nanoparticles.

The strength of the DC magnetic field may be less than 2,000 Oe (and greater than 0 Oe).

The frequency of the AC magnetic field may be 500 MHz to 6 GHz.

The pulse width of the AC magnetic field may be 0.3 sec. to 10 sec.

The strength of the AC magnetic field may be less than 10 Oe (and greater than 0 Oe).

The maximum value of the temperature change rate dT/dt of the magnetic nanoparticles may be increased by increasing the frequency of the AC magnetic field.

The maximum value of the temperature change rate dT/dt of the magnetic nanoparticles may be increased by increasing the strength of the AC magnetic field.

The magnetic nanoparticles may have a diameter greater than or equal to 5 nm and less than 500 nm.

The magnetic nanoparticles may be magnetic nanoparticles having a superparamagnetic structure or a single-domain structure, or magnetic nanoparticles having a magnetic vortex structure including a magnetic vortex core component, a horizontal magnetization component, and a spiral magnetization component.

The magnetic nanoparticles may include at least one of permalloy ($Ni_{80}Fe_{20}$), maghemite ($\gamma$-$Fe_2O_3$), magnetite ($\gamma$-$Fe_3O_4$), barium ferrite ($Ba_xFe_yO_z$, where x, y, and z are arbitrary numbers), $MnFe_2O_4$, $NiFe_2O_4$, $ZnFe_2O_4$, and $CoFe_2O_4$.

ADVANTAGEOUS EFFECTS

As described above, according to an embodiment of the present invention, a magnetic nanoparticle heating method capable of more efficiently generating heat from magnetic nanoparticles may be implemented.

According to an embodiment of the present invention, high heat may be generated by applying a low magnetic field.

According to an embodiment of the present invention, a low cost and a small size of an apparatus may be achieved.

According to an embodiment of the present invention, heat may be selectively and intensively generated in a specific treatment area for hyperthermia.

However, the scope of the present invention is not limited to the above effects.

DESCRIPTION OF THE DRAWINGS

FIG. 1 includes schematic diagrams of magnetic nanoparticles having superparamagnetism, a single magnetic domain, and a magnetic vortex structure, according to an embodiment of the present invention.

FIG. 2 includes schematic diagrams showing the magnetization orientation of magnetic nanoparticles due to the application of a first magnetic field, according to an embodiment of the present invention.

FIG. 3 includes graphs showing a change in resonance frequency of superparamagnetic nanoparticles and magnetic vortex nanoparticles based on a first magnetic field, according to an embodiment of the present invention.

FIG. 4 is a schematic diagram for describing an example of a method of applying a direct current (DC) magnetic field and an alternating current (AC) magnetic field to magnetic nanoparticles for a resonance of the magnetic nanoparticles, according to an embodiment of the present invention.

FIG. 5 includes graphs showing the resonance of magnetic nanoparticles based on a diameter of the magnetic nanoparticles when AC magnetic fields having different frequencies are applied, according to an embodiment of the present invention.

FIG. 6 is a schematic diagram of a magnetic nanoparticle heating apparatus according to an embodiment of the present invention.

FIG. 7 includes schematic diagrams of a magnet system according to an embodiment of the present invention.

FIG. 8 is a graph showing an amount of heat required to remove cancer cells based on a concentration of particles and a radius of a tumor, according to an embodiment of the present invention.

FIG. 9 includes graphs showing a temperature change rate of magnetic nanoparticles based on adjustment of a strength of a DC magnetic field, according to an embodiment of the present invention.

FIG. 10 includes graphs showing a temperature change rate of magnetic nanoparticles based on adjustment of a strength of a DC magnetic field and a frequency of an AC magnetic field, according to an embodiment of the present invention.

FIG. 11 is a graph showing a temperature change rate of magnetic nanoparticles based on adjustment of a strength of an AC magnetic field, according to an embodiment of the present invention.

FIG. 12 includes graphs showing a temperature change rate of magnetic nanoparticles based on adjustment of a pulse width of an AC magnetic field, according to an embodiment of the present invention.

<Description of Reference Numerals>

100: Magnetic nanoparticles
110: Magnetic vortex structure
120: Magnetic vortex core component
130: Horizontal magnetization component
140: Spiral magnetization component
200: Hyperthermia apparatus
210: Controller
230: Manipulator
250: Magnet system
251: Static magnetic field coil
253: Gradient coil
255: RF coil

MODE OF THE INVENTION

The following detailed description of the invention will be made with reference to the accompanying drawings illustrating specific embodiments of the invention by way of example. These embodiments will be described in sufficient detail such that the invention may be carried out by one of ordinary skill in the art. It should be understood that various embodiments of the invention are different but do not need to be mutually exclusive. For example, a specific shape, structure, or characteristic described herein in relation to an embodiment may be implemented as another embodiment without departing from the scope of the invention. In addition, it should be understood that positions or arrangements of individual elements in each disclosed embodiment may be changed without departing from the scope of the invention. Therefore, the following detailed description should not be construed as being restrictive and, if appropriately described, the scope of the invention is defined only by the appended claims and equivalents thereof. In the drawings, like reference numerals denote like functions, and lengths, areas, thicknesses, and shapes may be exaggerated for convenience's sake.

Hereinafter, the present invention will be described in detail by explaining embodiments of the invention with reference to the attached drawings, such that one of ordinary skill in the art may easily carry out the invention.

Although the following description is focused on magnetic nanoparticles having a single magnetic domain or a magnetic vortex structure, it is noted that the magnetic nanoparticles are not limited thereto and may include all magnetic nanoparticles capable of generating heat by using resonance.

[Magnetic Nanoparticles to be Heated]

Magnetic nanoparticles to be heated may include metal, e.g., iron (Fe), cobalt (Co), nickel (Ni), or an alloy thereof. The magnetic nanoparticles may include a superparamagnetic or ferromagnetic material. The magnetic nanoparticles may include, for example, permalloy ($Ni_{80}Fe_{20}$), maghemite ($\gamma$-$Fe_2O_3$), magnetite ($\gamma$-$Fe_3O_4$), barium ferrite ($Ba_xFe_yO_z$, where x, y, and z are arbitrary numbers), $MnFe_2O_4$, $NiFe_2O_4$, $ZnFe_2O_4$, or $CoFe_2O_4$. However, the material of the magnetic nanoparticles is not limited thereto.

When an external magnetic field having a certain strength is applied from the outside to magnetic nanoparticles, spins of the magnetic nanoparticles are oriented in the direction of the external magnetic field. When an alternating current (AC) or pulse magnetic field having a specific resonance frequency is applied to the magnetic nanoparticles oriented as described above, the magnetic nanoparticles exhibit a strong precession motion around the direction of the external magnetic field (or first magnetic field). The precession motion refers to a phenomenon in which a rotation axis of a rotating body rotates around an axis that does not move and, when an external magnetic field is applied to an electromagnetic field moving in a central force field, the magnetic moment of angular momentum rotates around the direction of the external direct current (DC) magnetic field as an axis.

The frequency of the precession motion is represented as shown in Equation 1.

[Equation 1]

$$f = L \cdot B$$

(where f denotes a frequency and B denotes the strength of a magnetic field)

Up to now, a material having a single spin has the value "L" in Equation 1 as a fixed constant value of 2.803 MHz/Oe, which is known as the Larmor frequency. Therefore, magnetic nanoparticles having a single magnetic domain, which also act as huge spin structures, have the Larmor frequency. The magnetic nanoparticles having a single magnetic domain may have a diameter greater than or equal to about 1 nm and less than about 40 nm.

However, when the diameter, shape, and/or material of the magnetic nanoparticles are changed, the magnetic nanoparticles do not act as single-domain particles and "L" in Equation 1 is no longer a constant value. That is, the magnetic nanoparticles do not have the Larmor frequency. In this specification, the magnetic nanoparticles not having the Larmor frequency are referred to as "magnetic nanoparticles having a magnetic vortex structure". For example, when the magnetic nanoparticles have a magnetic vortex structure, the magnetic nanoparticles have a resonance frequency changed based on a diameter thereof.

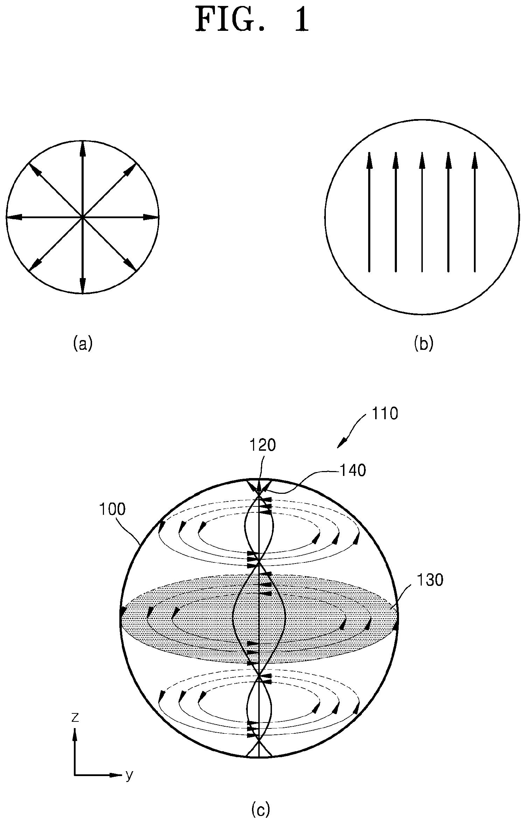

FIG. 1 includes schematic diagrams of magnetic nanoparticles 100 having superparamagnetism ((a) of FIG. 1), a single magnetic domain ((b) of FIG. 1), and a magnetic vortex structure 110 ((c) of FIG. 1), according to an embodiment of the present invention.

Magnetic nanoparticles may have superparamagnetism, a single magnetic domain, or the magnetic vortex structure 110. For example, when the magnetic nanoparticles include spherical permalloy ($Ni_{80}Fe_{20}$), the magnetic nanoparticles may be spheres having a diameter of several tens of nm to several hundreds of nm, and more specifically, a diameter greater than or equal to 5 nm and less than 500 nm. However, the diameter and the shape of the magnetic nanoparticles are merely examples and a case in which the magnetic nanoparticles have a non-spherical shape or a diameter greater than 500 nm may also be included in the scope of the present invention.

A case in which the magnetic nanoparticles 100 have the magnetic vortex structure 110 will now be described as an example with reference to (c) of FIG. 1. The magnetic vortex structure 110 may have a magnetic vortex core component 120, a horizontal magnetization component 130, and a spiral magnetization component 140.

The magnetic vortex core component 120 may penetrate the center of the magnetic nanoparticles 100, and have a +Z direction as a direction of magnetic force. The +Z direction may be determined by a direction of a magnetic field that the magnetic nanoparticles 100 have in advance or by a direction of an applied external magnetic field.

The horizontal magnetization component 130 may be positioned to rotate in a clockwise or counterclockwise direction with an orbit around the magnetic vortex core 120 as an axis. The horizontal magnetization component 130 may have the orbit in various shapes, e.g., a concentric or oval shape, based on the shape, material, and/or crystal orientation of the magnetic nanoparticles 100. The horizontal magnetization component 130 may form a certain angle from, for example, be perpendicular to, the magnetic vortex core 120. However, because the horizontal magnetization component 130 may have some magnetization component along or opposite to the direction of the magnetic vortex core 120 based on the properties, shape, and/or diameter of the magnetic nanoparticles 100, the magnetic vortex core 120 and the horizontal magnetization component 130 may not be perpendicular to each other. The horizontal magnetization component 130 may be present throughout the entire volume of the magnetic nanoparticles 100.

The spiral magnetization component 140 may be positioned adjacent to the magnetic vortex core 120, and be directed to the same direction as the magnetic vortex core 120. The spiral magnetization component 140 may be influenced by the horizontal magnetization component 130, and thus have a spirally rotating shape. Due to the spiral magnetization component 140, a magnetization direction in the magnetic nanoparticles 100 may be gradually changed from the magnetic vortex core 120 to the horizontal magnetization component 130. That is, the magnetization direction in the magnetic nanoparticles 100 may be gradually changed from a Z direction to a Y direction based on a position inside the magnetic nanoparticles 100.

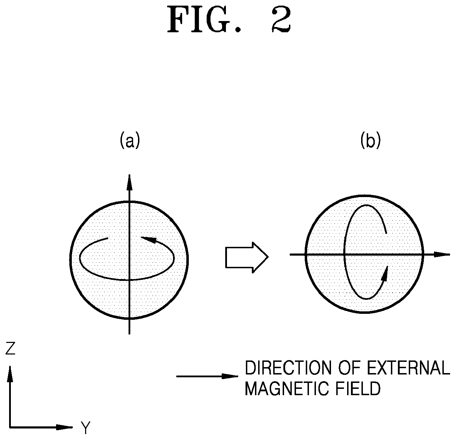

FIG. 2 includes schematic diagrams showing the magnetization orientation of magnetic nanoparticles due to the application of an external magnetic field (or first magnetic field), according to an embodiment of the present invention.

Referring to FIG. 2, a magnetization direction of the magnetic nanoparticles may be changed by the external magnetic field. In FIG. 2, +Z direction is used to indicate an average magnetization direction of the magnetic nanoparticles whereas a +Y direction is used to indicate a direction of a magnetic field applied from the outside to the magnetic nanoparticles, but the present invention is not limited to the directions. The +Z and +Y directions refer to different directions, and may or may not be perpendicular to each other.

(a) of FIG. 2 shows the magnetic nanoparticles before the external magnetic field (or first magnetic field) is applied, and the magnetic nanoparticles may have the +Z direction as the magnetization direction. That is, the average magnetization direction of the magnetic nanoparticles may be the +Z direction.

(b) of FIG. 2 shows the magnetic nanoparticles immediately after a weak external magnetic field is applied in the +Y direction. When the magnetic field is applied to the magnetic nanoparticles in the +Y direction different from the +Z direction which is the average magnetization direction of the magnetic nanoparticles, the internal magnetization orientation of the magnetic nanoparticles is directed to the +Y direction, and the magnetization in the +Y direction is gradually saturated in proportion to a strength of the external magnetic field.

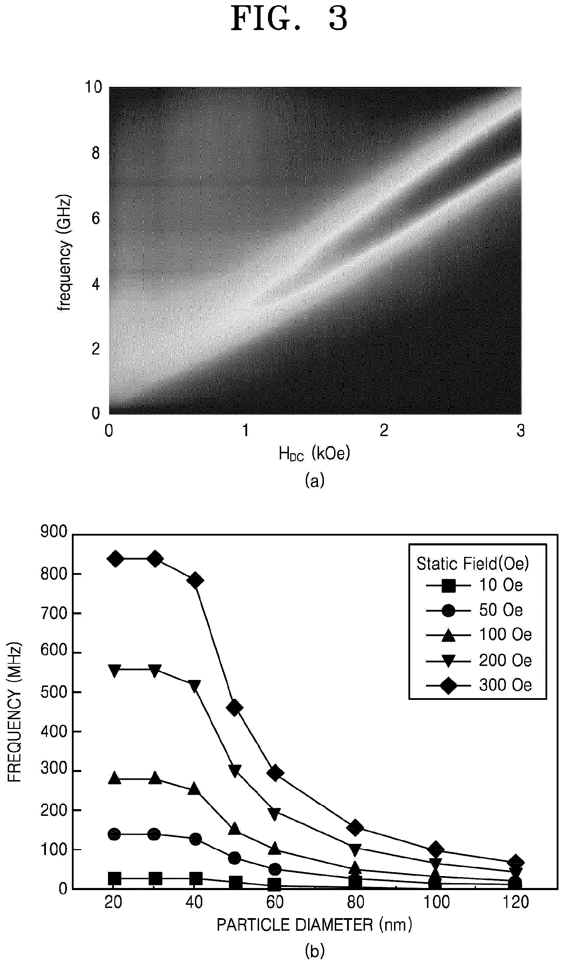

FIG. 3 includes graphs showing a change in resonance frequency of magnetic nanoparticles based on an external magnetic field (or first magnetic field), according to an embodiment of the present invention.

Referring to (a) of FIG. 3, iron oxide ($Fe_3O_4$) nanoparticles having a diameter of 15 nm have a superparamagnetic structure at room temperature. When an external static magnetic field (or first magnetic field) is applied thereto, the magnetic nanoparticles exhibit a precession motion along a direction of the magnetic field based on a strength of the external static magnetic field. At this time, the resonance frequency of the magnetic nanoparticles is proportional to the strength of the external magnetic field, and it is shown that this case corresponds to a case in which "L" in Equation 1 has a value similar to the constant value of 2.803 MHz/Oe which is the Larmor frequency.

Referring to (b) of FIG. 3, when an external static magnetic field (or first magnetic field) is applied, all spins of magnetic nanoparticles having a single magnetic domain and having a diameter greater than or equal to 20 nm and less than 40 nm exhibit a precession motion along a direction of the applied external magnetic field to change a magnetization direction. At this time, the resonance frequency of the magnetic nanoparticles is proportional to the external magnetic field, and it is shown that this case corresponds to a case in which "L" in Equation 1 has the constant value of 2.803 MHz/Oe which is the Larmor frequency.

Meanwhile, the resonance frequency of the magnetic nanoparticles having a magnetic vortex structure is reduced when the diameter thereof is increased. In addition, the resonance frequency is increased when the strength of the external magnetic field is increased. A reduction rate of the resonance frequency of the magnetic nanoparticles having a magnetic vortex structure and having a diameter greater than 40 nm is rapidly increased when the strength of the external magnetic field is increased.

Table 1 shows, as an embodiment, a resonance frequency based on a diameter of iron oxide ($Fe_3O_4$) or permalloy ($Ni_{80}Fe_{20}$) magnetic nanoparticles and a strength of an external static magnetic field.

[Table 1]

|  | 500 Oe | 1000 Oe | 1500 Oe | 2000 Oe | 2500 Oe |
|---|---|---|---|---|---|
| Superparamagnetic nanoparticles ($Fe_3O_4$) | | | | | |
| 15 nm | 2,270 MHz | 3,275 MHz | 4,605 MHz | 5,826 MHz | 7,283 MHz |
|  | 10 Oe | 50 Oe | 100 Oe | 200 Oe | 300 Oe |
| Single-domain magnetic nanoparticles ($Ni_{80}Fe_{20}$) | | | | | |
| 20 nm | 28 MHz | 140 MHz | 281 MHz | 562 MHz | 844 MHz |
| 30 nm | 28 MHz | 140 MHz | 281 MHz | 562 MHz | 844 MHz |
| Magnetic vortex nanoparticles ($Ni_{80}Fe_{20}$) | | | | | |
| 40 nm | 24 MHz | 124 MHz | 244 MHz | 516 MHz | 782 MHz |
| 60 nm | 10 MHz | 50 MHz | 95 MHz | 194 MHz | 294 MHz |
| 80 nm | 4 MHz | 24 MHz | 50 MHz | 102 MHz | 156 MHz |
| 100 nm | 2 MHz | 16 MHz | 32 MHz | 64 MHz | 98 MHz |
| 120 nm | 2 MHz | 12 MHz | 22 MHz | 44 MHz | 66 MHz |

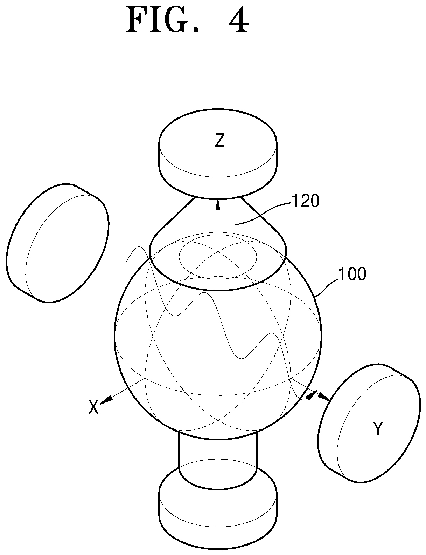

FIG. 4 is a schematic diagram for describing an example of a method of applying a DC magnetic field and an AC magnetic field to the magnetic nanoparticles 100 for a resonance of the magnetic nanoparticles 100, according to an embodiment of the present invention.

Referring to FIG. 4, a DC magnetic field is applied in a +Z direction of the magnetic nanoparticles 100 (i.e., a magnetization direction of the magnetic nanoparticles 100), and an AC magnetic field is applied in a +Y direction different from, for example, perpendicular to, the +Z direction. As shown in Table 1, a resonance frequency of the magnetic nanoparticles 100 may be determined based on a diameter of the magnetic nanoparticles 100 and a strength of the DC magnetic field. A strength of the AC magnetic field may be less than the strength of the DC magnetic field, and the behavior of the magnetic nanoparticles 100 is observed by changing a frequency of the AC magnetic field.

For example, the magnetic nanoparticles 100 are selected with a diameter of 30 nm and a diameter of 80 nm. The DC magnetic field applied in the Z direction is selected with a strength of about 100 Oe. The AC magnetic field applied in the Y direction is selected with a strength of about 10 Oe. The AC magnetic field is selected with a frequency of 281 MHz corresponding to a resonance frequency of the magnetic nanoparticles having a diameter of 30 nm, and a frequency of 50 MHz corresponding to a resonance frequency of the magnetic nanoparticles having a diameter of 80 nm.

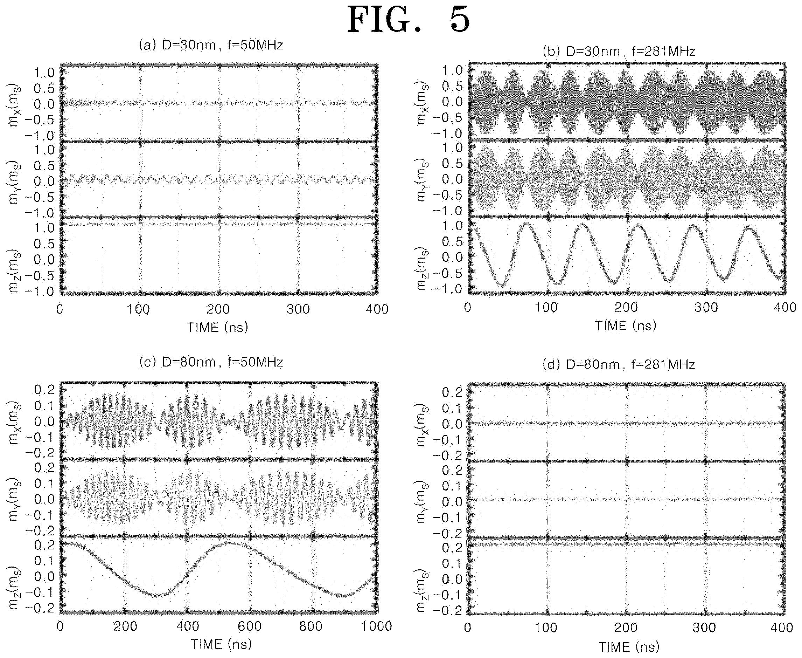

FIG. 5 includes graphs showing the resonance of magnetic nanoparticles based on a diameter of the magnetic nanoparticles when AC magnetic fields having different frequencies are applied. (a) and (b) of FIG. 5 correspond to magnetic nanoparticles having a diameter of 30 nm, and (c) and (d) of FIG. 5 correspond to magnetic nanoparticles having a diameter of 80 nm.

Referring to FIG. 5, the magnetic nanoparticles having a diameter of 30 nm exhibit no change when an AC magnetic field having a frequency of 50 MHz is applied (see (a)), but actively exhibit a strong precession motion and another motion such as magnetization reversal in response to application of an AC magnetic field having a frequency of 281 MHz corresponding to the resonance frequency thereof (see (b)).

The magnetic nanoparticles having a diameter of 80 nm exhibit no change when an AC magnetic field having a frequency of 281 MHz is applied (see (d)), but actively exhibit a strong precession motion and another motion such as magnetization reversal in response to application of an AC magnetic field having a frequency of 50 MHz corresponding to the resonance frequency thereof (see (c)).

That is, when a magnetic field having a resonance frequency of magnetic nanoparticles is applied, a motion of the magnetic nanoparticles, e.g., a precession motion, may be activated by the magnetic field.

Magnetic nanoparticles having superparamagnetism or a single magnetic domain have a different resonance frequency based on a first magnetic field (or DC magnetic field), and thus may generate heat when a second magnetic field (or AC magnetic field) corresponding to the resonance frequency is applied.

Magnetic nanoparticles having a magnetic vortex structure have a different resonance frequency based on a material, a diameter, or a first magnetic field (or DC magnetic field), and thus may selectively generate heat when a second magnetic field (or AC magnetic field) corresponding to the resonance frequency is applied.

[Magnetic Nanoparticle Heating Apparatus]

Embodiments to which the above-described magnetic nanoparticle heating method is applied will now be described. The present invention may be used in all fields requiring heating, and hyperthermia will be described below as an example.

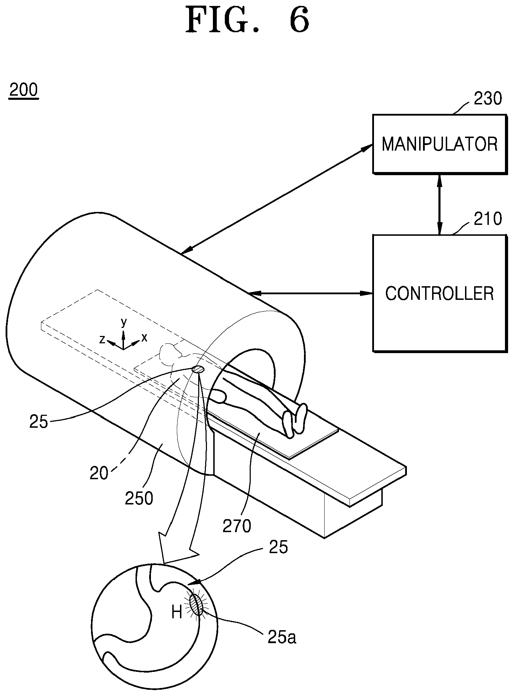

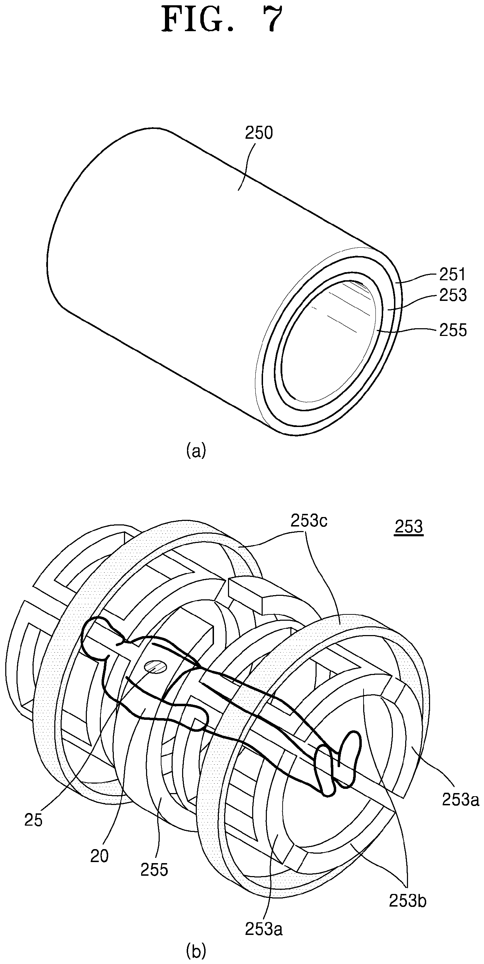

FIG. 6 is a schematic diagram of a magnetic nanoparticle heating apparatus 200 according to an embodiment of the present invention, and FIG. 7 includes schematic diagrams of a magnet system 250 according to an embodiment of the present invention.

The magnetic nanoparticles 100 having superparamagnetism, a single magnetic domain, or the magnetic vortex structure 110 may be provided to a treatment area 25 (or an affected area 25a). The provision of the magnetic nanoparticles 100 may be understood as injecting the magnetic nanoparticles 100 into a specific site of a patient (or a target object 20) having a disease and moving the target object 20 or a part of the target object 20 into the magnet system 250 of the magnetic hyperthermia apparatus 200. The magnetic nanoparticles 100 have a very fine diameter and thus may be uniformly distributed in the treatment area 25 (or the affected area 25a).

The hyperthermia apparatus 200 may include a controller 210, a manipulator 230, and the magnet system 250. The elements may form an integrated body without being physically separated as shown in FIG. 6.

The controller 210 may control a static magnetic field coil 251, a gradient coil 253, and a radio-frequency (RF) coil 255 of the magnet system 250. The controller 210 may control the magnet system 250 by analyzing an operation command received through the manipulator 230 from a user. The controller 210 may analyze an image signal received from the magnet system 250, generates an image signal corresponding thereto, and provide the generated image signal to a display of the manipulator 230.

The manipulator 230 may include an input device for receiving input from the user to control the hyperthermia apparatus 200, e.g., a keyboard or a mouse, and a display for displaying an image.

The target object (or patient) 20 may be moved into the magnet system 250 by a cradle 270. The cradle 270 may be omitted depending on a size of the hyperthermia apparatus 200, and the entirety or only a part of the target object 20 may be positioned in the magnet system 250.

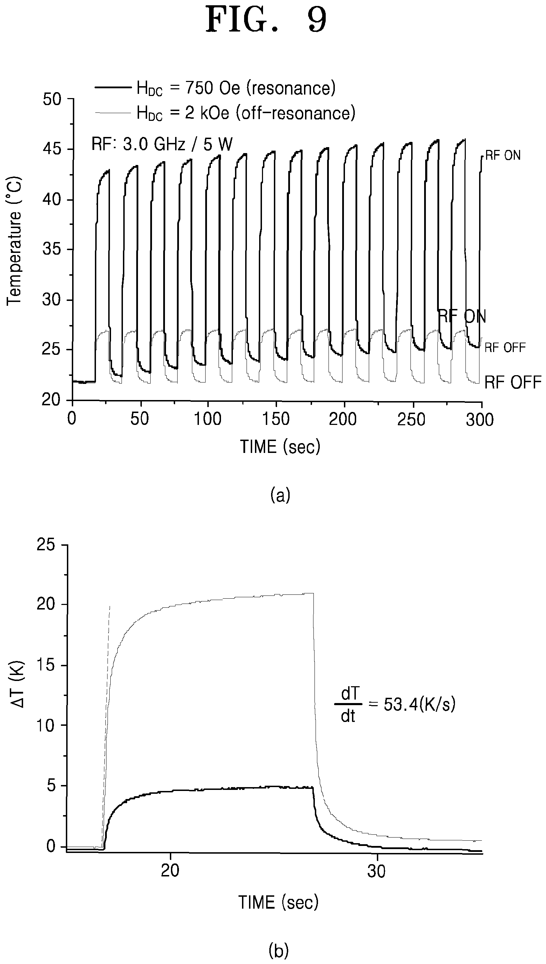

Referring to FIG. 9, the magnet system 250 may include the static magnetic field coil 251, the gradient coil 253, and the RF coil 255. The magnet system 250 may have a cylindrical shape and be coaxially provided, but is not limited thereto. The static magnetic field coil 251, the gradient coil 253, and the RF coil 255 may be provided in the above-mentioned order from the outside, and the RF coil 255 may have a hollow shape to accommodate the target object 20 therein.

The static magnetic field coil 251 may form a static magnetic field (or first magnetic field or DC magnetic field) in the magnet system 250. A direction of the static magnetic field may be parallel or perpendicular to a longitudinal direction of the target object 20, but is assumed in this specification as being parallel to the longitudinal direction of the target object 20.

The static magnetic field coil 251 may use a permanent magnet, a superconducting magnet, or an electromagnet. Because the magnetic nanoparticle heating method of the present invention does not require a high magnetic field of several T like existing apparatuses that apply only an AC magnetic field, the static magnetic field coil 251 is capable of forming a magnetic field of several mT to several hundreds of mT is sufficient. Therefore, equipment costs may be significantly reduced compared to the existing magnetic field forming apparatuses.

The gradient coil 253 may form a gradient field by generating a gradient in the static magnetic field. Because gradient fields for X, Y, and Z axes are all required to obtain 3-dimensional information, gradient coils 253a, 253b, and 253c may be provided for three axes.

When DC currents having opposite polarities flow in opposite directions through two Z-axis gradient coils 253c, a gradient field may be formed in a Z-axis direction. The Z-axis gradient coils 253c may be used to select a slice. Gradient fields by X-axis and Y-axis gradient coils 253a and 253b may be formed within the selected plane, and a frequency and a phase may be coded. As such, a spatial position of each spin may be coded (spatial coding).

The RF coil 255 may apply an RF pulse (or second magnetic field or AC magnetic field) for exciting the magnetic nanoparticles 100 in the target object 20. The RF coil 255 may include a transmitter coil for transmitting the RF pulse, and a receiver coil for receiving electromagnetic waves emitted from the excited magnetic nanoparticles 100.

When a DC magnetic field (or first magnetic field) is applied and an AC magnetic field (or second magnetic field) corresponding to a resonance frequency of the magnetic nanoparticles 100 is applied, heat may be generated from the magnetic nanoparticles 100 selectively activated based on a change in magnetization axis. As such, the heat may be transferred to treatment area 25 where the magnetic nanoparticles 100 are distributed.

For example, FIG. 6 shows that cancer cells 25a are present at an upper portion of a stomach 25. The magnetic nanoparticles 100 may be injected into and selectively and intensively distributed in the portion where the cancer cells 25a are present in the stomach 25. The heat H generated from the magnetic nanoparticles 100 may cause a temperature change of about 5K to about 15K in the treatment area 25 (or the cancer cells 25a) to kill the cancer cells 25a or a tumor of the treatment area 25. The heat H may be generated when charges are emitted or radiated from the magnetic nanoparticles 100 or when the magnetic nanoparticles 100 vibrate molecules of the treatment area 25.

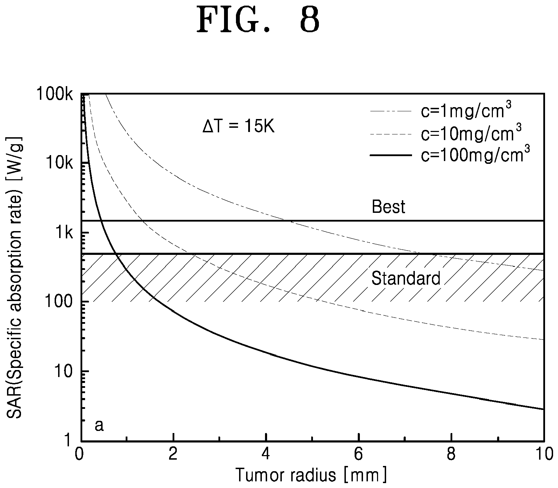

FIG. 8 is a graph showing an amount of heat required to remove a tumor based on a concentration of particles and a radius of the tumor, according to an embodiment of the present invention.

A temperature change $\Delta T$, which is caused when heat H generated from particles is transferred to a tumor or cells, follows Equation 2. In general, an ideal temperature change $\Delta T$ required to remove the tumor (or the cancer cells 25a) is 15K.

[Equation 2]

$$\Delta T = SAR \cdot c \cdot R^2 / (3\lambda)$$

(Where SAR, which is an abbreviation for specific absorption rate and is replaceable with specific heating power, indicates an amount of heat generated per sec. and per weight of particles under an AC magnetic field, c indicates a concentration of particles adsorbed to cells, R indicates a radius of a tumor or cells, and $\lambda$ indicates a thermal conductivity which is $\lambda=0.64$ WK$^{-1}$m$^{-1}$ for tissue.)

Referring to FIG. 8, to achieve a certain temperature change $\Delta T$, it may be considered to adsorb the particles (i.e., the magnetic nanoparticles 100) at a high concentration c or increase the amount of heat SAR. In particular, for effectively treat tumors, treatment of tumors having a radius R of 10 mm or more also needs to be enabled. Because it is currently not easy to adsorb the particles to cancer cells at a high concentration, the lower the concentration c, the better. Eventually, according to Equation 2, increasing the amount of heat SAR may be a major factor to control the temperature change. According to FIG. 8, an amount of heat SAR of at least 0.1 kW/g, and more specifically, an amount of heat SAR of 2 kW/g, is required to treat a tumor having a radius R of 10 mm or more by adsorbing the particles at a concentration of 1 mg/cm$^3$. Although existing technology may generate heat of only up to several tens of W/g to several hundreds of W/g by applying a magnetic field having a strength of several hundreds of Oe, the present invention may sufficiently generate heat higher than 2 kW/g by controlling each factor as described below.

[Magnetic Nanoparticle Heating Method]

Meanwhile, to be effectively used for hyperthermia, the generation of a large amount of heat SAR from magnetic nanoparticles is critical, but a generation of sufficient heat for treatment within a short time is considered more critical. When a long time is taken to generate heat, the heat is spread to surrounding normal cells without being concentrated on target cells (or a tumor) for hyperthermia and thus the therapeutic effect is significantly reduced.

Therefore, the present invention proposes a method capable of increasing a temperature change rate dT/dt of magnetic nanoparticles. Specifically, the present invention proposes a method capable of increasing a temperature change rate dT/dt of magnetic nanoparticles to at least 10 K/s or more by adjusting at least one of a strength of a DC magnetic field, a frequency of an AC magnetic field, a strength of the AC magnetic field, and a pulse width of the AC magnetic field when the magnetic fields are applied to heat the magnetic nanoparticles.

A magnetic nanoparticle heating method according to an embodiment of the present invention includes (a) providing the magnetic nanoparticles 100, (b) applying a DC magnetic field to the magnetic nanoparticles 100, and (c) applying an AC magnetic field to the magnetic nanoparticles 100. When the magnetic nanoparticles 100 generate heat in step (c), a heating rate may be adjusted by adjusting at least one of a strength of the DC magnetic field, a frequency of the AC magnetic field, a strength of the AC magnetic field, and a pulse width of the AC magnetic field.

Initially, in step (a), the magnetic nanoparticles 100 may be provided. For example, the magnetic nanoparticles 100 of the present invention may be provided by moving the magnetic nanoparticles 100 into the magnet system 250 to apply a magnetic field to the magnetic nanoparticles 100 (see FIG. 6).

Subsequently, in step (b), the DC magnetic field may be applied to the magnetic nanoparticles 100. In particular, the DC magnetic field may be applied to make the magnetic nanoparticles 100 have a resonance frequency. As described above in relation to FIG. 5, the resonance frequency of the magnetic nanoparticles 100 having superparamagnetism or a single magnetic domain is changed based on the DC magnetic field, and the magnetic nanoparticles 100 having the magnetic vortex structure 110 may have a resonance frequency changed based on a diameter thereof.

The DC magnetic field may be formed by the static magnetic field coil 251 (see FIG. 7) of the magnet system 250 to be described below. The strength of the DC magnetic field may be less than 2,000 Oe (and greater than 0 Oe), and the DC magnetic field may be in a range of several tens of Oe to several hundreds of Oe, e.g., a range greater than or equal to 10 Oe and less than 300 Oe, when the magnetic nanoparticles include spherical permalloy ($Ni_{80}Fe_{20}$). However, the above-mentioned range of the DC magnetic field is an example and is not limited thereto. As described above in relation to FIG. 3, an allowed strength of the first magnetic field may be increased when the diameter of the magnetic nanoparticles 100 is increased.

The resonance frequency of the magnetic nanoparticles 100 may be changed based on a material, the diameter, and/or a shape of the magnetic nanoparticles 100.

Subsequently, in step (c), the AC magnetic field may be applied to the magnetic nanoparticles 100. In particular, the AC magnetic field having a frequency equal to the resonance frequency of the magnetic nanoparticles 100 may be applied to the magnetic nanoparticles 100. For example, the frequency of the AC magnetic field may be 500 MHz to 6 GHz, and the strength of the AC magnetic field may be less than 10 Oe (and greater than 0 Oe).

The AC magnetic field (or pulse magnetic field) may be understood as an RF pulse formed by the RF coil 255 (see FIG. 7) of the magnet system 250. The AC magnetic field may be applied in a direction having a certain angle from a direction in which the DC magnetic field is applied, and the direction having a certain angle may be a perpendicular direction.

As described above in relation to FIG. 5, when the AC magnetic field is applied, the magnetic nanoparticles 100 having superparamagnetism, a single magnetic domain, or the magnetic vortex structure 110 actively exhibit a strong precession motion and another motion such as magnetization reversal and a change in magnetization axis occurs.

Subsequently, heat may be generated from the magnetic nanoparticles 100 based on the change in the magnetization axis. The heat may be generated when charges are emitted or radiated from the magnetic nanoparticles 100 or when the magnetic nanoparticles 100 vibrate molecules of a surrounding material or a target material to be heated.

As an existing hyperthermia method, a method of generating thermal fluctuations by applying only an AC magnetic field to magnetic nanoparticles and releasing the application of the AC magnetic field to use the heat generated by relaxation has been proposed. This method is based on a principle of generating heat by using energy due to magnetic hysteresis loss of the magnetic nanoparticles (width of a hysteresis loop), or generating heat by using friction with a surrounding medium or other particles due to relaxation of the magnetic moment of the nanoparticles (Brownian relaxation). However, according to the existing method, because magnetization reversal needs to be caused by applying only an AC magnetic field, a very strong magnetic field of several hundreds of Oe or more needs to be applied, which leads to a high cost and a large size of an apparatus.

On the other hand, according to the magnetic nanoparticle heating method of the present invention, because heat may be generated based on the resonance of magnetic nanoparticles by applying a DC magnetic field and an AC magnetic field, heat may be efficiently generated using only a relatively weak magnetic field of several tens of Oe, which directly leads to a low cost and a small size of an apparatus. In addition, a resonance frequency of the magnetic nanoparticles may be controlled based on the DC magnetic field applied to the magnetic nanoparticles (see Table 1), and an amount of heat may be freely controlled by controlling the resonance frequency. When applied to hyperthermia, the resonance frequency of the magnetic nanoparticles may be controlled within a range that is not harmful to human bodies, and thus heat ideal for hyperthermia may be generated.

[Adjustment of Temperature Change Rate of Magnetic Nanoparticles]

A method of obtaining a temperature change rate and an amount of heat, which are proper to use the magnetic nanoparticle heating method from various viewpoints, will now be described.

FIG. 9 includes graphs showing a temperature change rate of magnetic nanoparticles based on adjustment of a strength of a DC magnetic field, according to an embodiment of the present invention. While a DC magnetic field $H_{DC}$ is being applied at strengths of 750 Oe and 2,000 Oe, an AC magnetic field of 3.0 GHz and 5W is repeatedly applied on/off at intervals of 10 sec.

Referring to (a) of FIG. 9, it is shown that temperature changes for $H_{DC}$ = 750 Oe are significantly different from those for $H_{DC}$ = 2,000 Oe. This temperature difference is caused by resonance of the magnetic nanoparticles, and it is shown that $H_{DC}$ = 750 Oe is a condition for causing resonance when the AC magnetic field of 3 GHz is applied. The temperature may be rapidly increased by about 20 °C for about 1 sec. in an early stage of application of the AC magnetic field. For $H_{DC}$ = 2,000 Oe corresponding to off resonance, the temperature is increased by only about 5 °C or less, and this temperature increase may be regarded as an extra temperature increase due to dielectric heating or Joule heating.

Referring to (b) of FIG. 9, a temperature increase rate for early 1 sec. under a resonance condition is dT/dt = 53.4 K/s. This value is about 50 times greater than a temperature increase rate (1 K/s or less) exhibited by existing hyperthermia methods using only an AC magnetic field. When converted into the SAR value described above in relation to FIG. 8, the temperature increase rate corresponds to about 1.3 kW/g, and satisfies a minimum value of 0.1 kW/g which is required to treat tumors having a radius R of 10 mm or more by absorbing particles at a concentration of 1 mg/cm$^3$.

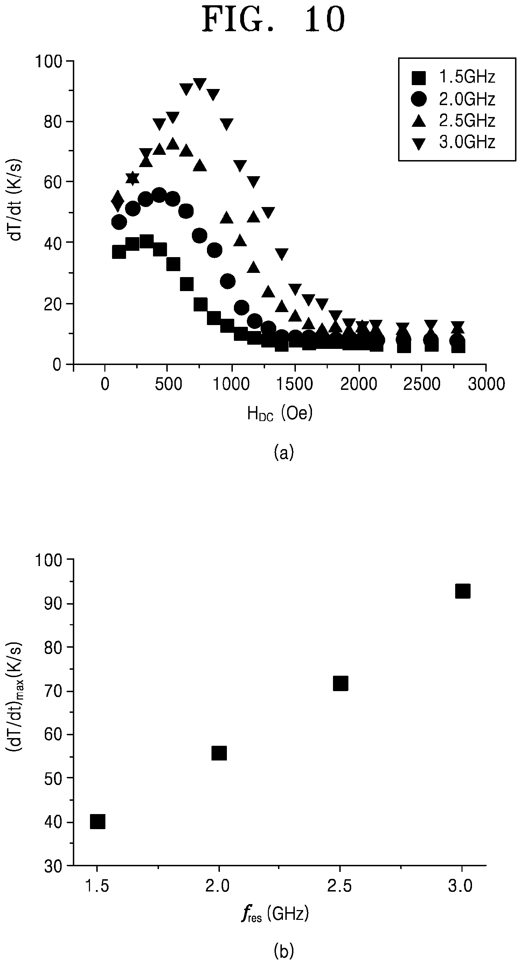

FIG. 10 includes graphs showing a temperature change rate of magnetic nanoparticles based on adjustment of a strength of a DC magnetic field and a frequency of an AC magnetic field, according to an embodiment of the present invention. AC magnetic fields of 1.5 GHz, 2.0 GHz, 2.5 GHz, and 3.0 GHz having a strength of 2.37 Oe are applied on/off at intervals of 1 sec., and a DC magnetic field is applied gradually from 0 Oe to 3,000 Oe.

Referring to (a) of FIG. 10, it is shown that each plot has a maximum value of a temperature change rate dT/dt due to resonance. The AC magnetic field of 3.0 GHz exhibits a temperature change rate dT/dt of about 92 K/s under a condition of $H_{DC}$ = 750 Oe. Thereafter, under a condition of $H_{DC}$ ≥ 2,000 Oe, a constant value of about 13 K/s, which is not changed based on the strength of the DC magnetic field, is exhibited. For the frequencies other than 3.0 GHz, the temperature change rate dT/dt due to resonance is maximized at a strength of the DC magnetic field $H_{DC}$ appropriate for each frequency.

Referring to (b) of FIG. 10, it is shown that the maximum value of the temperature change rate dT/dt at each frequency is increased in proportion to the frequency of the AC magnetic field. When converted into SAR values, values of about 40 K/s at 1.5 GHz, about 56 K/s at 2.0 GHz, about 72 K/s at 2.5 GHz, and about 93 K/s at 3.0 GHz correspond to about 1.0 kW/g, about 1.4 kW/g, about 1.8 kW/g, and about 2.3 kW/g, respectively, and satisfy not only a minimum value of 0.1 kW/g which is required to treat tumors having a radius R of 10 mm or more by absorbing particles at a concentration of 1 mg/cm$^3$, but also a value of 2 kW/g which is ideal for tumor treatment.

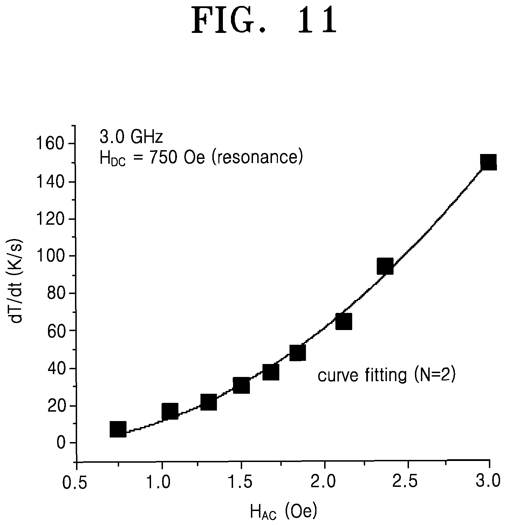

FIG. 11 is a graph showing a temperature change rate of magnetic nanoparticles based on adjustment of a strength of an AC magnetic field, according to an embodiment of the present invention. While a DC magnetic field $H_{DC}$ is being applied at a strength of 750 Oe and an AC magnetic field is being applied at a frequency of 3.0 GHz, a strength of the AC magnetic field is gradually increased.

Referring to FIG. 11, a temperature change rate dT/dt exhibits about 7.35 K/s when $H_{AC}$ = 0.75 Oe, and exhibits about 149.64 K/s when $H_{AC}$ is gradually increased to $H_{AC}$ = 3.0 Oe. It is shown that dT/dt is increased in quadratic proportion to the strength of $H_{AC}$ overall.

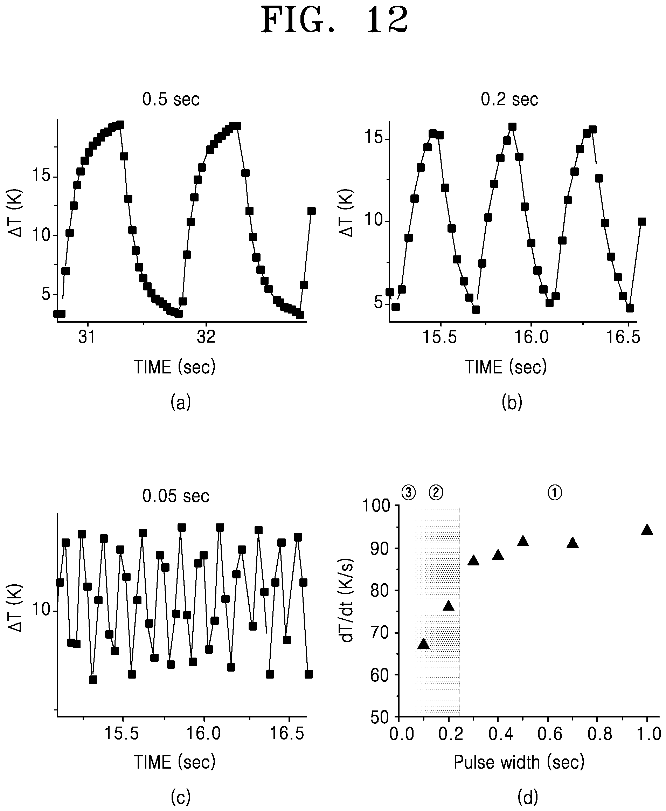

FIG. 12 includes graphs showing a temperature change rate of magnetic nanoparticles based on adjustment of a pulse width of an AC magnetic field, according to an embodiment of the present invention. While a DC magnetic field $H_{DC}$ is being applied at a strength of 750 Oe and an AC magnetic field is being applied at a frequency of 3.0 GHz and a strength of 2.73 Oe, dT/dt is measured by reducing a pulse width of the AC magnetic field.

Referring to (a) of FIG. 12, when the AC magnetic field is applied on/off at intervals of 0.5 sec., a repeated temperature increase/reduction is shown, and the value of dT/dt is not significantly changed. This shape is maintained in a period in which the pulse width of the AC magnetic field is reduced from 1 sec. to 0.3 sec.

Referring to (b) of FIG. 12, when the AC magnetic field is applied on/off at intervals of 0.2 sec., because the temperature is increased again due to re-application of the AC magnetic field before the temperature reaches an initial point, it is shown that the value of dT/dt is reduced.

Referring to (c) of FIG. 12, dT/dt is exhibited irregularly in a period of several tens of ms less than 0.1 sec., which is because of the limitation of a thermal resolution of a thermal imaging camera, and it is predicted that behavior similar to (b) of FIG. 12 will be shown.

(d) of FIG. 12 collectively shows data of the periods of (a) to (c) of FIG. 12, and a repeated temperature increase/reduction is shown regardless of the pulse width of the AC magnetic field in period ① (corresponding to (a) of FIG. 12).

Based on the results of FIGS. 9 to 12, it is shown that a temperature change rate and an amount of heat may be freely controlled based on a strength of a DC magnetic field, a frequency of an AC magnetic field, a strength of the AC magnetic field, and a pulse width of the AC magnetic field, and that maximum values of the temperature change rate and the amount of heat are significantly greater than those of existing hyperthermia methods.

Although the existing hyperthermia methods may achieve a temperature change rate of only up to 1 K/s by applying an AC magnetic field having a strength of several hundreds of Oe corresponding to 100 Oe to 300 Oe, the present invention may implement a temperature change rate greater than 10 K/s, and more specifically, a temperature change rate greater than 50 K/s, by using an AC magnetic field having a strength less than 10 Oe and a DC magnetic field having a strength less than 2,000 Oe. As such, heat ideal for hyperthermia may be generated using a low-cost small apparatus, and be effectively transferred to a treatment area inside a human body by using low-concentration magnetic nanoparticles. In addition, because a resonance frequency of the magnetic nanoparticles may be controlled based on the DC magnetic field and an amount of heat may also be controlled based on the resonance frequency, a temperature may be adjusted considering the characteristics of the treatment area.

While the present invention has been particularly shown and described with reference to embodiments thereof, it will be understood by one of ordinary skill in the art that various changes in form and details may be made therein without departing from the scope of the present invention as defined by the following claims.

**Claims**

1. A magnetic nanoparticle heating method comprising:

   (a) providing magnetic nanoparticles;
   (b) applying a direct current (DC) magnetic field to the magnetic nanoparticles; and
   (c) applying an alternating current (AC) magnetic field to the magnetic nanoparticles 100,

   wherein a temperature change rate dT/dt of the magnetic nanoparticles is increased to at least 10 K/s or more by adjusting at least one of a strength of the DC magnetic field, a frequency of the AC magnetic field, a strength of the AC magnetic field, and a pulse width of the AC magnetic field.

2. The magnetic nanoparticle heating method of claim 1, wherein step (b) comprises applying the DC magnetic field to make the magnetic nanoparticles have a resonance frequency, and step (c) comprises applying the AC magnetic field having a frequency equal to the resonance frequency of the magnetic nanoparticles, to exhibit a maximum value of the temperature change rate dT/dt of the magnetic nanoparticles.

3. The magnetic nanoparticle heating method of claim 1, wherein the strength of the DC magnetic field is less than 2,000 Oe (and greater than 0 Oe).

4. The magnetic nanoparticle heating method of claim 1, wherein the frequency of the AC magnetic field is 500 MHz to 6 GHz.

5. The magnetic nanoparticle heating method of claim 1, wherein the pulse width of the AC magnetic field is 0.3 sec. to 10 sec.

6. The magnetic nanoparticle heating method of claim 1, wherein the strength of the AC magnetic field is less than 10 Oe (and greater than 0 Oe).

7. The magnetic nanoparticle heating method of claim 2, wherein the maximum value of the temperature change rate dT/dt of the magnetic nanoparticles is increased by increasing the frequency of the AC magnetic field.

8. The magnetic nanoparticle heating method of claim 2, wherein the maximum value of the temperature change rate dT/dt of the magnetic nanoparticles is increased by increasing the strength of the AC magnetic field.

9. The magnetic nanoparticle heating method of claim 1, wherein the magnetic nanoparticles have a diameter greater than or equal to 5 nm and less than 500 nm.

**10.** The magnetic nanoparticle heating method of claim 9, wherein the magnetic nanoparticles are magnetic nanoparticles having a superparamagnetic structure or a single-domain structure, or
magnetic nanoparticles having a magnetic vortex structure comprising a magnetic vortex core component, a horizontal magnetization component, and a spiral magnetization component.

**11.** The magnetic nanoparticle heating method of claim 1, wherein the magnetic nanoparticles comprise at least one of permalloy ($Ni_{80}Fe_{20}$), maghemite (y-$Fe_2O_3$), magnetite ($\gamma$-$Fe_3O_4$), barium ferrite ($Ba_xFe_yO_z$, where x, y, and z are arbitrary numbers), $MnFe_2O_4$, $NiFe_2O_4$, $ZnFe_2O_4$, and $CoFe_2O_4$.

# FIG. 1

(a)

(b)

(c)

# FIG. 2

(a)          (b)

Z

Y

DIRECTION OF EXTERNAL
MAGNETIC FIELD

# FIG. 3

(a)

(b)

# FIG. 4

# FIG. 5

(a) D=30nm, f=50MHz

(b) D=30nm, f=281MHz

(c) D=80nm, f=50MHz

(d) D=80nm, f=281MHz

EP 4 104 898 A1

# FIG. 6

200

230 MANIPULATOR

210 CONTROLLER

25

20

250

270

25

H

25a

# FIG. 7

(a)

(b)

# FIG. 8

# FIG. 9

(a)

$$\frac{dT}{dt} = 53.4(K/s)$$

(b)

# FIG. 10

(a)

(b)

# FIG. 11

# FIG. 12

**0.5 sec**

(a)

**0.2 sec**

(b)

**0.05 sec**

(c)

(d)

<div align="center">**INTERNATIONAL SEARCH REPORT**</div>

| International application No. |
| --- |
| **PCT/KR2020/010888** |

**A. CLASSIFICATION OF SUBJECT MATTER**

*A61N 2/02*(2006.01)i; *A61N 2/00*(2006.01)i; *A61K 41/00*(2006.01)i; **H01F 1/00**(2006.01)i

According to International Patent Classification (IPC) or to both national classification and IPC

**B. FIELDS SEARCHED**

Minimum documentation searched (classification system followed by classification symbols)

A61N 2/02; A61B 5/055; A61K 33/26; A61K 9/14; A61K 9/16; A61N 2/00; A61N 2/04; G01R 33/20; A61K 41/00; H01F 1/00

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 자성 나노 입자 발열(magnetic nanoparticle heating ), 직류 자기장(direct magnetic field), 교류 자기장(alternating magnetic field), 온도 변화 속도(temperature changing rate), 공명 주파수(resonant frequency), 자화 구조(magnifying structure)

**C. DOCUMENTS CONSIDERED TO BE RELEVANT**

| Category* | Citation of document, with indication, where appropriate, of the relevant passages | Relevant to claim No. |
| --- | --- | --- |
| X | WO 2017-188725 A1 (UNIV SEOUL NAT R & DB FOUND) 02 November 2017. See paragraphs [0023], [0056], [0084], [0093], [0112], [0119] and [0121]; claim 1; and table 1. | 1-11 |
| X | KR 10-2014-0032756 A (SEOUL NATIONAL UNIVERSITY R&DB FOUNDATION) 17 March 2014. See paragraphs [0022]-[0023], [0027]-[0035], [0089] and [0094]; and figure 1. | 1,3-6,9-11 |
| A | BAE, Seongtae et al. Applications of NiFe2O4 nanoparticles for a hyperthermia agent in biomedicine. Applied Physics Letters. 2006, vol. 89, Article No. 252503, pp. 252503-1-202503-3. See entire document. | 1-11 |
| A | KR 10-2000-0034772 A (PARAGON MEDICAL LIMITED) 26 June 2000. See entire document. | 1-11 |

✓ Further documents are listed in the continuation of Box C.          ✓ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **23 November 2020** | **24 November 2020** |

| Name and mailing address of the ISA/KR | Authorized officer |
| --- | --- |
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.

**PCT/KR2020/010888**

**C.      DOCUMENTS CONSIDERED TO BE RELEVANT**

| Category* | Citation of document, with indication, where appropriate, of the relevant passages | Relevant to claim No. |
|---|---|---|
| A | KR 10-1623116 B1 (SEOUL NATIONAL UNIVERSITY R&DB FOUNDATION) 23 May 2016. See entire document. | 1-11 |
| X | LEE, Jae-Hyeok et al. Ferromagnetic resonance effect in superparamagnetic iron oxide nanoparticles and its application for hyperthermia. 한국자기학회 학술연구발표회 논문개요집 (Proceedings of The Korean Magnetics Society Conference). November 2019, vol. 29, no. 2, p. 64. See page 64. (* This document is a known document declaring exceptions to lack of novelty by the applicant.) | 1,3-6,9-11 |

Form PCT/ISA/210 (second sheet) (July 2019)

**INTERNATIONAL SEARCH REPORT**
**Information on patent family members**

International application No.

**PCT/KR2020/010888**

| Patent document cited in search report | | | Publication date (day/month/year) | Patent family member(s) | | | Publication date (day/month/year) |
|---|---|---|---|---|---|---|---|
| WO | 2017-188725 | A1 | 02 November 2017 | | None | | |
| KR | 10-2014-0032756 | A | 17 March 2014 | EP | 2893920 | A1 | 15 July 2015 |
| | | | | EP | 2893920 | B1 | 19 December 2018 |
| | | | | US | 9336934 | B2 | 10 May 2016 |
| | | | | US | 2015-0213931 | A1 | 30 July 2015 |
| | | | | WO | 2014-038829 | A1 | 13 March 2014 |
| | | | | KR | 10-1409296 | B1 | 24 June 2014 |
| KR | 10-2000-0034772 | A | 26 June 2000 | AT | 320283 | T | 15 April 2006 |
| | | | | AU | 2627797 | A | 05 December 1997 |
| | | | | CA | 2253963 | A1 | 20 November 1997 |
| | | | | CN | 1218415 | A | 02 June 1999 |
| | | | | EP | 0952873 | B1 | 15 March 2006 |
| | | | | EP | 0952873 | A1 | 03 November 1999 |
| | | | | ES | 2260789 | T3 | 01 November 2006 |
| | | | | JP | 2000-503879 | A | 04 April 2000 |
| | | | | US | 6167313 | A | 26 December 2000 |
| | | | | US | 6565887 | B1 | 20 May 2003 |
| | | | | WO | 97-43005 | A1 | 20 November 1997 |
| KR | 10-1623116 | B1 | 23 May 2016 | | None | | |

Form PCT/ISA/210 (patent family annex) (July 2019)